# FASCICULE DE BREVET EUROPEEN

(11) **EP 0 297 411 B1**
(45) Date de publication et mention de la délivrance du brevet: **13.01.1993**
(21) Numéro de dépôt: 88109930.3
(22) Date de dépôt: 22.06.1988
(51) Int. Cl.: A61F 13/46, A61F 13/50

(54) **Matelas absorbant perfectionné, notamment pour des produits d'hygiène, et procédé de fabrication en continu de tels matelas**
Absorbierende Matte, insbesondere für Hygieneartikel und Verfahren zu ihrer kontinuierlichen Herstellung
Absorbant mat, particularly for hygienic articles and method of its continuous manufacture

(30) Priorité: 26.06.1987 FR 8709096
(43) Date de publication de la demande: 04.01.1989
(73) Titulaire: PEAUDOUCE, 59126 Linselles (FR)
(72) Inventeur: Koczab, Jean-Pierre, F-59910 Bondues (FR)
(74) Mandataire: Casalonga, Axel

(56) Documents cités:
- DE-B- 1 163 491
- FR-A- 2 263 887
- FR-A- 2 562 394
- US-A- 4 413 995
- US-A- 4 590 114

## Description

La présente invention se rapporte à un matelas absorbant, notamment pour des produits d'hygiène tels que des couches-culottes, comprenant au moins une nappe de fibres, ainsi qu'à un procédé de fabrication en continu de tels matelas absorbants.

De façon usuelle, les matelas absorbants utilisés pour les produits d'hygiène tels que les couches-culottes, garnitures pour incontinents, etc. sont constitués par une ou plusieurs nappes de fibres, notamment de pulpe de cellulose défibrée (dite fluff), recouverte(s) ou non sur une ou deux faces par un feuillet externe de ouate de cellulose ou d'un produit analogue. Pour améliorer la cohésion d'un tel matelas, il est déjà connu de soumettre le matelas à un gaufrage consistant à comprimer le matelas par endroits espacés, suivant un dessin répétitif.

Toutefois, la cohésion ainsi obtenue est souvent insuffisante.

Par ailleurs, suivant la demande de brevet français FR-A-2,592,896 au nom de la demanderesse, un matelas absorbant peut être formé à partir d'au moins d'une nappe de fibres d'une longueur au moins égale à l'épaisseur de la nappe, la nappe étant liée par aiguilletage ou un procédé analogue faisant appel aux fibres de la nappe elle-même pour le liage de cette dernière. Un tel matelas présente une bonne cohésion, mais ne peut pas être fabriqué directement sur des machines usuelles de production de couches-culottes. Par ailleurs, du fait de l'aiguilletage, la vitesse de fabrication est limitée. Il en résulte un prix de revient relativement élevé.

Depuis peu de temps, on incorpore par ailleurs à des matelas en matériau fibreux des particules de superabsorbant généralement constituées par des polymères insolubles dans l'eau, capables d'absorber plusieurs fois (au moins quinze fois) leur poids en liquide.

Lorsque les particules de superabsorbant absorbent du liquide, elles gonflent et se transforment en une masse gélatineuse. Ces particules gélatineuses, lorsqu'elles sont proches les unes des autres, ont tendance à s'agglomérer et peuvent alors former une couche empêchant le passage ultérieur du liquide ce qui réduit la capacité d'absorption. Par ailleurs, compte tenu de leur faible taille à l'état sec, ces particules des matière superabsorbante ont tendance à s'accumuler et à s'échapper du matelas, notamment aux bords de ce dernier.

La demande de brevet français précitée FR-A-2,592,896 apporte déjà, par l'aiguilletage du matelas, une solution à ces problèmes de fixation et de retenue des particules de superabsorbant dans le matelas. Toutefois, comme déjà mentionné, cet aiguilletage implique des vitesses de production réduites et ne permet pas une fabrication du matelas sur une machine usuelle de production de couches-culottes.

La présente invention a pour objet un matelas absorbant comprenant au moins une nappe de fibres, le matelas se distinguant, tout en présentant une bonne capacité d'absorption, par une bonne cohésion et par un prix de revient réduit. L'invention a également pour objet un matelas absorbant comprenant au moins une nappe de fibres et contenant des particules de superabsorbant, ce matelas se distinguant par une bonne fixation et retenue des particules de superabsorbant à l'intérieur du matelas. L'invention a par ailleurs pour objet un procédé de fabrication en continu de matelas absorbants, procédé qui procure des matelas de bonne cohésion et peut être mis en oeuvre à une vitesse de production élevée sur une machine usuelle de fabrication de couches-culottes. L'invention a enfin pour objet un procédé de fabrication en continu de matelas absorbants contenant des particules de superabsorbant.

Sur le matelas absorbant conforme à l'invention, notamment pour des produits d'hygiène tels que des couches-culottes, comprenant au moins une nappe de fibres, une partie au moins des fibres est constituée par des fibres thermofusibles réparties sur toute l'épaisseur de la nappe et les fibres du matelas sont liées par endroits espacés, au travers du matelas, sur toute l'épaisseur de ce dernier, par compression du matelas et fusion desdites fibres thermofusibles auxdits endroits.

Cette liaison des fibres du matelas procure à l'ensemble du matelas une cohésion nettement supérieure à celle obtenue par un gaufrage à froid, sans affecter de façon sensible la capacité d'absorption du matelas.

Dans le cas d'un matelas absorbant contenant des particules de superabsorbant mélangées aux fibres, les fibres du matelas sont avantageusement liées suivant un dessin à mailles fermées.

Cette liaison suivant un dessin à mailles fermées assure un emprisonnement des particules de superabsorbant à l'intérieur des zones délimitées par les mailles, ce qui réduit dans une très large mesure les risques d'accumulation des particules et d'échappement des particules sur les bords du matelas.

Notamment pour améliorer la cohésion et solidité de surface du matelas, ce dernier peut comprendre, en outre, sur au moins une face de la nappe de fibres, un feuillet extérieur de matière fibreuse également lié par la compression du matelas et la fusion des fibres thermofusibles de la nappe de fibres.

Ce feuillet extérieur peut être par exemple constitué de ouate de cellulose ou également d'un non-tissé.

Suivant le procédé conforme à l'invention de fabrication en continu de matelas absorbants comprenant au moins une nappe de fibres, on forme une nappe continue de fibres dont une partie au moins des fibres est constituée par des fibres thermofusibles réparties sur toute l'épaisseur de la nappe et on calandre ladite nappe à chaud de manière à comprimer le matelas et à faire fusionner les fibres thermofusibles par endroits espacés pour lier les fibres du matelas au travers du matelas auxdits endroits.

La nappe de fibres constituée en partie au moins par des fibres thermofusibles et contenant, le cas échéant, des particules de superabsorbant peut être réalisée sans difficulté sur un poste de nappage usuel, tel qu'utilisé par exemple sur une machine de fabrication de couches-culottes, et le calandrage à chaud de cette nappe peut être effectué à la même vitesse élevée que les autres opérations de fabrication de couches-culottes.

Lors de l'incorporation de particules de superabsorbant aux fibres de la nappe, on calandre le matelas de manière à comprimer la matelas et à faire fusionner les fibres thermofusibles suivant un dessin à mailles fermées.

De préférence, on recouvre la nappe de fibres ou la nappe de fibres et de particules superabsorbant sur une face au moins d'un feuillet externe de matière fibreuse avant de soumettre la nappe avec ledit feuillet au calandrage à chaud.

En se référant aux dessins schématiques annexés, on va décrire ci-après plus en détail un mode de réalisation illustratif et non limitatif de l'objet de l'invention; sur les dessins :
la figure 1 est une vue en plan d'une partie d'un matelas absorbant conforme à l'invention;
la figure 2 est une coupe à plus grande échelle suivant II-II de la figure 1;
la figure 3 représente très schématiquement les différentes phases du procédé de fabrication d'un matelas absorbant suivant les figures 1 et 2;
la figure 4 est une section partielle de deux rouleaux de calandrage utilisés dans le procédé de fabrication suivant la figure 3.

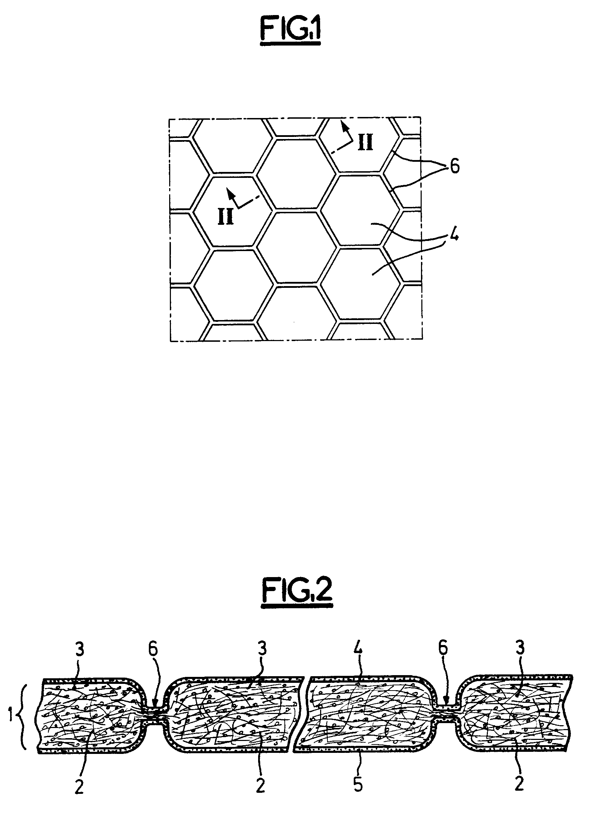

Le matelas absorbant illustré par les figures 1 et 2 est composé d'une nappe 1 formée de matière fibreuse 2 et de particules 3 de superabsorbant incorporées à la matière fibreuse 2, ainsi que de deux feuillets externes 4 et 5 perméables aux liquides, recouvrant la nappe 1 sur ses deux faces.

La matière fibreuse 2 est constituée, en partie au moins, par des fibres thermofusibles, c'est-à-dire des fibres de matière thermoplastique. Lorsque la matière fibreuse 2 comprend à la fois des fibres thermofusibles et des fibres non thermofusibles, les fibres des deux types sont mélangées. Les feuillets externes 4, 5 peuvent être constitués, l'un ou l'autre ou les deux, par exemple par un voile de ouate de cellulose ou un voile de non-tissé.

Le matelas dans son ensemble, c'est-à-dire la nappe 1 de matière fibreuse 2 à laquelle sont incorporées les particules de superabsorbant, et les deux feuillets extérieurs 4, 5, est soumis à un calandrage à chaud "partiel", c'est-à-dire n'intéressant qu'une faible partie de la surface du matelas. Ce calandrage ne porte sur le matelas qu'en des endroits espacés 6. A ces endroits espacés 6, le calandrage à chaud provoque une compression du matelas et une fusion des fibres thermofusibles contenues dans la nappe 1. Sous l'effet de cette compression à chaud, il se produit, au travers du matelas, une liaison des fibres intéressant toutes les fibres de la nappe 1, c'està-dire à la fois les fibres thermofusibles et les éventuelles fibres non fusibles. Par ailleurs, cette liaison produite par la fusion des fibres thermofusibles de la nappe 1 intéresse également les deux feuillets externes 4, 5, compte tenu de la nature fibreuse de ces feuillets.

Le résultat de ce calandrage à chaud consiste donc d'une part en une amélioration de la cohésion de la nappe 1 en elle-même et d'autre part en une liaison des feuillets extérieurs 4, 5 à la nappe 1.

Par ailleurs, dans le mode de réalisation représenté, ce calaudrage à chaud aux endroits référencés 6 est effectué suivant un dessin répétitif à mailles fermées, en l'occurrence à mailles hexagonales jointives. Un tel calandrage subdivise donc le matelas en une multitude de cellules ou alvéoles chacune délimitée par les deux feuillets extérieurs 4, 5 et une ligne de calandrage 6 fermée, hexagonale, de sorte que les particules de superabsorbant 3 se trouvent emprisonnées dans lesdites cellules et ne risquent pas de se déplacer à l'intérieur de la nappe 1, voire de s'échapper de la nappe 1.

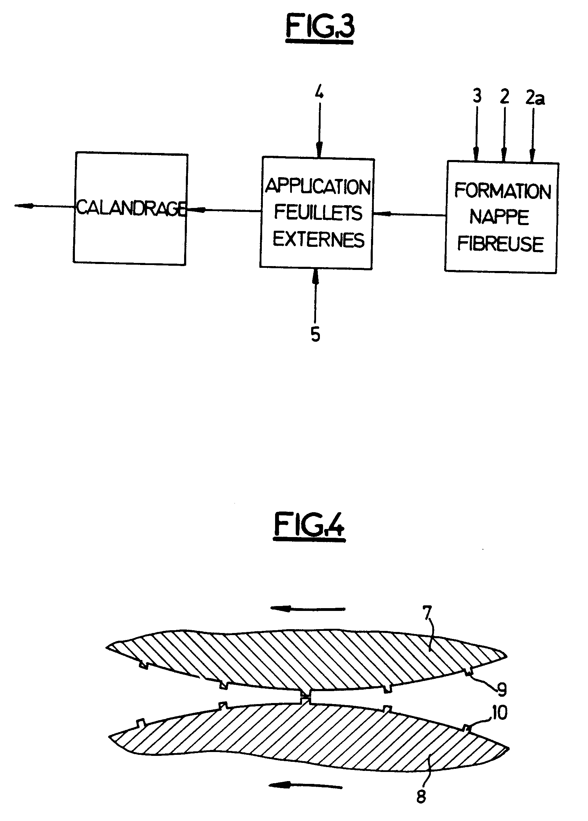

La fabrication d'un matelas absorbant suivant les figures 1 et 2 peut se faire suivant un procédé dont le schéma est illustré par la figure 3.

Selon la figure 3, on forme d'abord, à partir de fibres 2 thermofusibles et éventuellement d'autres fibres 2a non thermofusibles une nappe fibreuse à laquelle les particules de superabsorbant 3 sont incorporées. Cette formation de la nappe fibreuse contenant du superabsorbant peut se faire suivant la technique usuelle de dépôt de fibres en suspension dans de l'air, dite "air-laying" (nappe dite "air-felt").

On applique ensuite, sur la nappe fibreuse ainsi formée, les deux feuillets externes 4 et 5 avant de soumettre le matelas (nappe fibreuse recouverte des feuillets externes) à un calandrage à chaud partiel.

La figure 4 représente schématiquement une paire de rouleaux 7 et 8 susceptibles d'être utilisés pour ce calandrage du matelas absorbant. Les deux rouleaux 7, 8 qui sont couplés portent, sur leur surface latérale, le même dessin 9, 10 en relief, de telle manière que lorsque les deux rouleaux 7, 8 tournent en synchronisme dans les sens indiqués par les flèches, leurs dessins 9, 10 en relief défilent en coïncidence l'un en face de l'autre à l'endroit de l'interstice le plus étroit entre les deux rouleaux 7, 8. Les deux rouleaux 7, 8 sont chauffés par des moyens usuels, non représentés, de manière que les reliefs 9 et 10 soient portés à une température suffisante pour provoquer, dans le matelas passant entre les deux rouleaux 7, 8, une fusion des fibres thermofusibles contenues dans le matelas.

Les fibres 2 constituant la nappe fibreuse 1 peuvent comprendre uniquement des fibres thermofusibles, par exemple des fibres de polypropylène, de polyéthylène, de polyamide ou de polyester. Il est cependant possible également d'utiliser un mélange de fibres thermofusibles et de fibres non thermofusibles, par exemple des fibres de viscose, du fluff de cellulose, etc. Par ailleurs, il serait également possible d'utiliser des fibres du type bis-composant formées par exemple d'une âme et d'une enveloppe ou deux secteurs adjacents en deux matières différentes ayant des points de fusion différents, par exemple une âme de polyester et une enveloppe de polyéthylène.

Toutes les fibres utilisées peuvent être de préférence des fibres courtes susceptibles d'être amenées sous la forme d'une nappe suivant la technique usuelle dite "air-laying".

Le superabsorbant susceptible d'être incorporé à la nappe de fibres du matelas conforme à l'invention peut être constitué par tous les superabsorbants du commerce, par exemple des polymères à base d'acide polyacrylique ou des dérivés d'amidon ou de cellulose obtenus par greffage, ce superabsorbant pouvant se présenter sous forme de poudre, de fibres, etc. De tels superabsorbants sont par exemple commercialisés sous la marque "Lucquabsorb" par la Société BASF, RFA, ou sous la marque "Aquakeep" par la Société SEITETSU KAGAKU, Japon.

Suivant un exemple du matelas absorbant conforme à l'invention, le matelas est constitué d'une nappe fibreuse de 78 g/m² formée de 80% de fibres de viscose et de 20% de fibres de polypropylène. Des grains de superabsorbant sont incorporés à cette nappe fibreuse à raison de 39 g/m². La nappe fibreuse est recouverte sur chacune de ces faces par un pli de ouate de cellulose.

L'ensemble du matelas ainsi constitué est soumis à calandrage à chaud avec un dessin à mailles fermées de forme hexagonale de 10 mm de côté, la largeur des lignes de calandrage étant de 1 mm. Ce calandrage conduit à une subdivision du matelas en cellules dans lesquelles des grains de superabsorbant sont retenus et fixés de façon sûre.

Il va de soi que le mode de réalisation représenté et décrit n'a été donné qu'à titre d'exemple illustratif et que de nombreuses modifications et variantes sont possibles dans le cadre de l'invention.

Ainsi, lorsque la nappe fibreuse du matelas ne contient pas de particules de superabsorbant, il est possible de procéder au calandrage suivant un dessin quelconque procurant simplement un calandrage par endroits espacés.

En cas d'incorporation de particules de superabsorbant à la nappe fibreuse du matelas, il est possible de choisir un calandrage à mailles fermées autre que de forme hexagonale, par exemple à mailles carrées.

Afin que le calandrage affecte le moins possible les propriétés d'absorption du matelas, il est avantageux que ce calandrage concerne moins de 10% et de préférence moins de 5% de la surface du matelas. Malgré que le calandrage ne concerne qu'une si faible proportion de la surface du matelas, l'effet de consolidation qu'il procure au matelas est bien suffisant pour la plupart des application en articles d'hygiène. De même, il procure un effet suffisant de fixation et de retenue des particules de superabsorbant dans le matelas.

Par ailleurs, il serait également possible de réaliser, suivant l'invention, un matelas à partir de plus d'une nappe, les nappes étant réunies entre elles par le calandrage à chaud. Dans ce cas, les nappes peuvent, par exemple, contenir des fibres différentes ou des mélanges différents de fibres, de même qu'elles peuvent contenir des proportions différentes de superabsorbants éventuellement différents, par exemple des superabsorbants ayant des vitesses d'absorption différentes.

## Revendications

1. Matelas absorbant, notamment pour des produits d'hygiène tels que des couches-culottes, comprenant au moins une nappe de fibres, comprimée par endroits et des particules de superabsorbant, caractérisé par le fait qu'une partie au moins des fibres (2) est constituée par des fibres thermofusibles réparties sur toute l'épaisseur de la nappe (1), que les particules de superabsorbant sont mélangées aux libres de la nappe, qu'un feuillet extérieur (4, 5) de matière fibreuse est disposé sur au moins l'une des deux faces de la nappe, et que les libres du matelas, y compris celles dudit feuillet extérieur (4, 5), sont liées par endroits espacés au travers du matelas, sur toute l'épaisseur de ce dernier, par compression à chaud du matelas et fusion des fibres thermofusibles de la nappe suivant un dessin à mailles fermées subdivisant le matelas en cellules assurant la fixation et la retenue des particules de superabsorbant dans le matelas.

2. Matelas suivant la revendication 1, caractérisé par le fait que la compression du matelas affecte moins de 10% de préférence moins de 5% de la surface du matelas.

3. Matelas suivant la revendication 1 ou 2, caractérisé par le fait que le matelas contient des particules de superabsorbant dans des proportions supérieures à 1:4 et de préférence sensiblement égales à 1:2 par rapport au poids des fibres de la nappe.

4. Matelas suivant l'une quelconque des revendications précédentes, caractérisé par le fait que l'un au moins desdits feuillets extérieurs est constitué de ouate de cellulose.

5. Matelas suivant l'une quelconque des revendications précédentes, caractérisé par le fait que l'un au moins desdits feuillets extérieurs est constitué d'un voile de non-tissé.

6. Procédé de fabrication en continu de matelas absorbants, notamment pour des produits d'hygiène tels que des couches-culottes, comprenant au moins une nappe de fibres comprimée par endroits et des particules de superabsorbant, caractérisé par le fait qu'on recouvre une nappe continue formée de fibres dont une partie au moins est constituée par des fibres thermofusibles réparties sur toute l'épaisseur de la nappe, et de particules de superabsorbant mélangées auxdites fibres, sur au moins l'une de ses deux faces d'un feuillet extérieur de matière fibreuse et qu'on calandre à chaud le matelas ainsi formé de manière à comprimer le matelas et faire fusionner les fibres thermofusibles par endroits espacés pour lier, suivant un dessin à mailles fermées, les fibres de la nappe au travers de cette dernière et pour lier les fibres dudit feuillet extérieur à la nappe.

## Claims

1. An absorbent pad, in particular for sanitary products such as nappy-pants, comprising at least one layer of fibres, which is compressed at intervals, and particles of superabsorbent material, characterised in that at least one portion of the fibres (2) is formed by thermofusible fibres distributed over the entire thickness of the layer (1), in that the particles of superabsorbent material are mixed with the fibres of the layer, in that a thin outer sheet (4, 5) of fibrous material is disposed on at least one of the two surfaces of the layer, and in that the fibres of the pad, including those of said thin outer sheet (4, 5), are bound at spaced locations across the pad, over the entire thickness thereof, by hot compression of the pad and fusion of the thermofusible fibres of the layer in accordance with a closed-mesh pattern, dividing the pad up into cells ensuring the fixing and retention of the particles of superabsorbent material in the pad.

2. A pad according to claim 1, characterised in that the compression of the pad affects less than 10 %, preferably less than 5 %, of the surface of the pad.

3. A pad according to claim 1 or 2, characterised in that the pad contains particles of superabsorbent material in proportions which are higher than 1:4 and, preferably, substantially equal to 1:2 in relation to the weight of the fibres of the layer.

4. A pad according to any one of the preceding claims, characterised in that at least one of said thin outer sheets is composed of cellulose wadding.

5. A pad according to any one of the preceding claims, characterised in that at least one of said thin outer sheets is composed of a covering of non-woven material.

6. A method for the continuous production of absorbent pads, in particular for sanitary products such as nappy-pants, comprising at least one layer of fibres, which is compressed at intervals, and particles of superabsorbent material, characterised in that a continuous layer composed of fibres, of which at least one portion is formed by thermofusible fibres distributed over the entire thickness of the layer, and of particles of superabsorbent material mixed with said fibres, is covered on at least of its two surfaces with a thin outer sheet of fibrous material, and in that the pad thus formed is hot-calendered so as to compress the pad and to cause the thermofusible fibres to fuse at spaced locations so as to bind, in accordance with a closed-mesh pattern, the fibres of the layer across the latter and so as to bind the fibres of said thin outer sheet to the layer.

## Patentansprüche

1. Absorbierende Matte, insbesondere für Hygieneartikel, wie Slip-Einlagen, welche mindestens ein Faservlies, das stellenweise zusammengedrückt ist, und Partikel aus einem superabsorbierenden Material enthält, dadurch **gekennzeichnet,** dass mindestens ein Teil der Fasern (2) aus in der Wärme schmelzbaren Fasern zusammengesetzt ist, die über die gesamte Dicke des Vlieses (1) verteilt sind, dass die Partikel aus superabsorbierendem Material mit den Fasern des Vlieses vermischt sind, dass ein Aussenblatt (4, 5) aus Fasermaterial auf mindestens einer der beiden Seiten des Vlieses angeordnet ist, und dass die Mattenfasern, eingeschlossen diejenigen des genannten Aussenblattes (4, 5) an durch Zwischenräume getrennten stellen quer durch die Matte, über die gesamte Dicke der letzteren, durch in der Wärme durchgeführte Kompression der Matte und zusammenschmelzen der wärmeschmelzbaren Fasern des Vlieses gemäss eines Musters in geschlossenen Maschen verbunden werden, wobei die Matte in Zellen unterteilt wird, was die Fixierung und das Zurückhalten der superabsorbierenden Partikel in der Matte gewährleistet.

2. Matte gemäss Anspruch 1, dadurch **gekennzeichnet,** dass die Kompression der Matte weniger als 10 %, vorzugsweise weniger als 5 %, der Oberfläche der Matte betrifft.

3. Matte gemäss Anspruch 1 oder 2, dadurch **gekennzeichnet,** dass die Matte Partikel aus superabsorbierendem Material in Mengenverhältnissen oberhalb 1:4, vorzugsweise ziemlich genau bei 1:2, bezogen auf das Gewicht der Vliesstoffasern, enthält.

4. Matte gemäss jedem der vorhergehenden Ansprüche, dadurch **gekennzeichnet,** dass mindestens eines der genannten Aussenblätter aus Zellulosewatte zusammengesetzt ist.

5. Matte gemäss jedem der vorhergehenden Ansprüche, dadurch **gekennzeichnet,** dass mindestens eines der genannten Aussenblätter aus einem Flor aus Vliesstoff zusammengesetzt ist.

6. Verfahren zur kontinuierlichen Herstellung von absorbierenden Matten, insbesondere für Hygieneartikel, wie Slip-Einlagen, welche mindestens ein stellenweise zusammengedrücktes Faservlies und Partikel aus superabsorbierendem Material enthalten, dadurch **gekennzeichnet,** dass man ein kontinuierliches Vlies, das aus Fasern, die zumindest teilweise aus wärmeschmelzbaren Fasern zusammengesetzt sind, die über die gesamte Dicke des Vlieses verteilt sind, und aus Partikeln aus superabsorbierendem Material gebildet ist, die mit den genannten Fasern vermischt sind, auf mindestens einer seiner beiden Seiten mit einem Aussenblatt aus Fasermaterial bedeckt und die so gebildete Matte in der Wärme kalandert, und zwar so, dass man die Matte komprimiert und die wärmeschmelzbaren Fasern an durch Zwischenräume getrennten Stellen zusammenschmelzen lässt, um, gemäss einem Muster in geschlossenen Maschen, die Fasern des Vlieses quer durch das letztere zu verbinden und die Fasern des genannten Aussenblattes mit dem Vlies zu verbinden.
